# EUROPEAN PATENT APPLICATION

(11) **EP 4 011 412 A1**
(43) Date of publication of application: **15.06.2022**
(21) Application number: 20213210.6
(22) Date of filing: 10.12.2020
(51) Int. Cl.: A61L 27/36, A61F 2/24

(54) **CARDIAC VALVE PROSTHESIS**

(71) Applicant: GrOwnValve GmbH, 10435 Berlin (DE)
(72) Inventor: SCHMITT, Boris, 10435 Berlin (DE)
(74) Representative: Maikowski & Ninnemann Patentanwälte Partnerschaft mbB

(57) **Abstract**

The present invention relates to a cardiac valve prosthesis, obtainable by a method comprising the following steps: a) providing human or animal body tissue, b) shaping the body tissue in a shaping process to give the body tissue a shape and size of a cardiac valve, c) fixation and stabilization of the body tissue by a cross-linking agent, thereby preserving the shape given to the body tissue by the shaping process and thus obtaining a cardiac valve prosthesis.

## Description

The present invention relates to a cardiac valve prosthesis according to the preamble of claim 1 and to a medical use of such a cardiac valve prosthesis according to the preamble of claim 14.

WO 2004/047620 A2 describes a process of fixing tissue with a solution comprising a phenolic tannin. This international patent application further describes a process for replacing a damaged cardiac valve by implanting a bioprosthetic heart valve comprising fixed tissue comprising elastin cross-linked with a tannic acid cross-linking agent. The bioprostheses disclosed in this international patent application comprise, besides fixed tissue comprising elastin cross-linked with a tannic acid cross-linking agent, a support material attached to the fixed tissue.

WO 2004/113431 A1 relates to the use of a secoiridoid-containing substance as non-toxic cross-linking agent for the cross-linking of biopolymers, such as polypeptides polysaccharides.

WO 2006/115733 A2 relates to methods and products for the treatment of connective tissue weakened due to destruction of tissue architecture, in particular due to an elastin degradation. The treatment agents employ certain unique properties of phenolic compounds for a reduction of elastin degradation.

It is an object of the present invention to provide an improved cardiac valve prosthesis that can be made from human or animal body tissue, is stable and can be produced in a particularly simple manner.

This object is achieved with a cardiac prosthesis having the features of claim 1. Such a cardiac valve prosthesis is or can be obtained by a method comprising the steps explained in the following. First, human or animal body tissue is provided. Then, the body tissue is shaped in a shaping process to give the body tissue a shape and a size of a cardiac valve. Afterwards, the shaped body tissue is fixed and stabilized by a cross-linking agent. This results in preserving the shape given to the body tissue by the shaping process. As a result, a cardiac valve prosthesis is obtained.

The term "body tissue" comprises connective tissue, muscular tissue, nervous tissue, epithelial tissue, fascial tissue, peritoneal tissue and cardiac tissue. While these kinds of tissue also contain liquid components, they can referred to as solid body tissues. The term "body tissue" does explicitly not comprise liquid tissues like blood or lymph.

In an embodiment, the term "body tissue" does not encompass natural cardiac valve tissue.

In an embodiment, the animal body tissue originates from a rodent or a non-human mammal.

In contrast to prior art techniques, according to which cross-linking agents are merely used to decrease the susceptibility of the used body tissue with respect to biodegradability, the method applied for manufacturing the claimed cardiac valve prosthesis makes use of a shaping process in which a desired shape and size, namely the shape and size of a cardiac valve, is given to the body tissue. The cross-linking agent then preserves this given shape. There is no indication in the prior art that a cross-linking agent could be used for preserving a given shape. Rather, to give an example, WO 2004/047620 A2 discloses a bioprosthesis that comprises on the one hand tissue fixed with a cross-linking agent but, on the on the hand, additionally a support material attached to the fixed tissue. Thus, the prior art teaches that the used cross-linking agents can be used for modifying the chemical structure of the treated body tissue. However, the prior art does not teach that a given shape of body tissue can be preserved by applying a cross-linking agent.

In contrast, the cardiac valve prosthesis according to the presently claimed invention is a self-contained cardiac valve prosthesis that does not need nor contains any further support material or support structures. Rather, it is free of additional support material or support structures. The cardiac valve prosthesis may be implanted in form of a cardiac valve prosthesis arrangement comprising the cardiac valve prosthesis and a carrier (e.g., a stent) connected to the cardiac valve prosthesis. For connecting the cardiac valve prosthesis to the carrier, the cardiac valve prosthesis can be sewn to the carrier. The carrier is, however, not necessary for structurally supporting the cardiac prosthesis. It rather serves for keeping the cardiac valve prosthesis in place in an implanted state and for allowing proper functioning of the cardiac valve prosthesis. In an embodiment, the shaping process is a process in which positive or negative pressure is applied to the body tissue. To give an example, the body tissue can be inserted into a mold and deformed by applying pressure, e.g., by a suction process. It then adapts the shape of the mold.

In an embodiment, the shaping process is a deep drawing process, e.g., a deep drawing process using an individually shaped last.

In an embodiment, the cross-linking does not only preserve the given shape of the body tissue, but also serves for stabilizing the structure of the extracellular matrix of the body tissue, wherein the extracellular matrix comprises glycosaminoglycans, collagen and elastin.

In an embodiment, the cross-linking agent comprises at least one or is a secoiridoid corresponding to the following general formula (I):

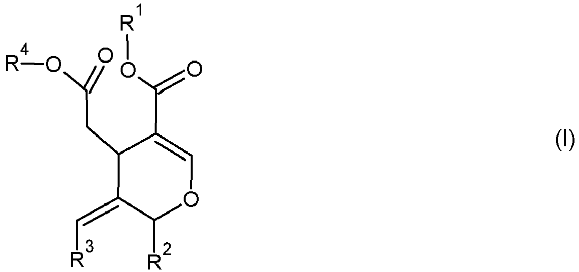

Thereby,
- R¹ and R³: denote independently of each other and independently of other residues in the compound H or CH₃,
- R⁴: denotes independently of other residues in the compound H or a residue having a structure according to general formula (II): 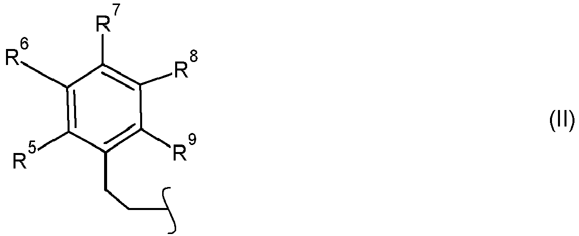
wherein
- R⁵, R⁶, R⁷, R⁸, R⁹: denote independently of each other and independently of other residues in the compound H or OH,
- R²: denotes independently of other residues in the compound H, OH or a residue having a structure according to general formula (III): 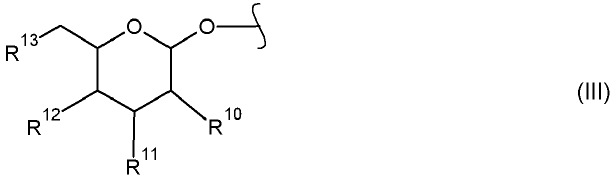
wherein
- R¹⁰, R¹¹, R¹², R¹³: denote independently of each other and independently of other residues in the compound H or OH.

In case that R² corresponds to formula (III), the linkage between residue R² and the neighboring heterocycle of the compound is effected by the bond extending away from the oxygen atom of residue R² that is bound to the heterocycle of residue R² so that a structure of the following formula (VI) results:

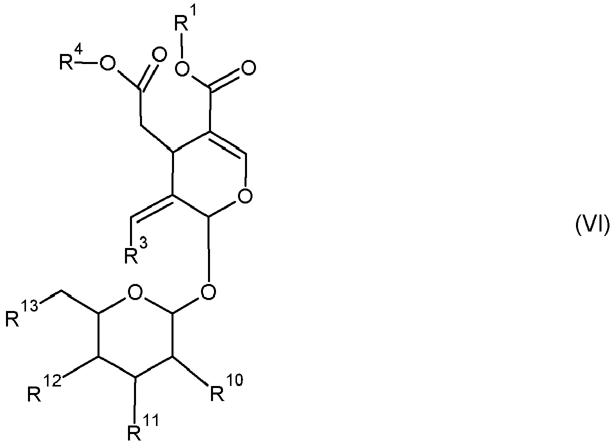

In an embodiment, the residues of the structure according to formula (I) have a conformation according to the following formula (IV):

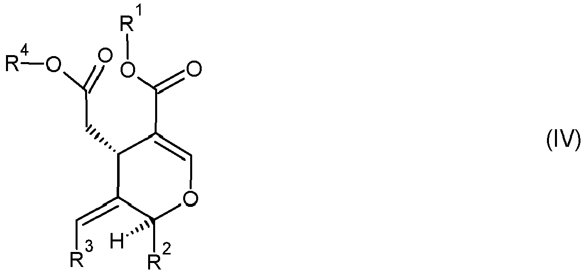

In an embodiment, the residue R² has a structure according to general formula (III) and has a conformation according to the following formula (V):

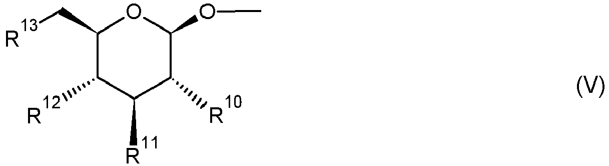

By a combination of the latter two embodiments, the following structure according to general formula (VII) results:

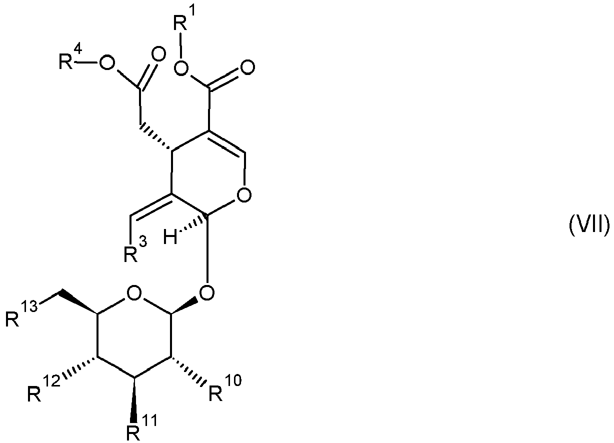

In an embodiment, at least two of residues R⁵, R⁶, R⁷, R⁸, and R⁹ denote OH.

In an embodiment, residues R⁵, R⁶, and R⁹ denote H and residues R⁷ and R⁸ denote OH.

In an embodiment, residues R¹ and R³ denote CH₃.

In an embodiment, residue R² denotes OH. Then, the cross-linking agent comprises or corresponds to the following general formulae (VIII) or (IX):

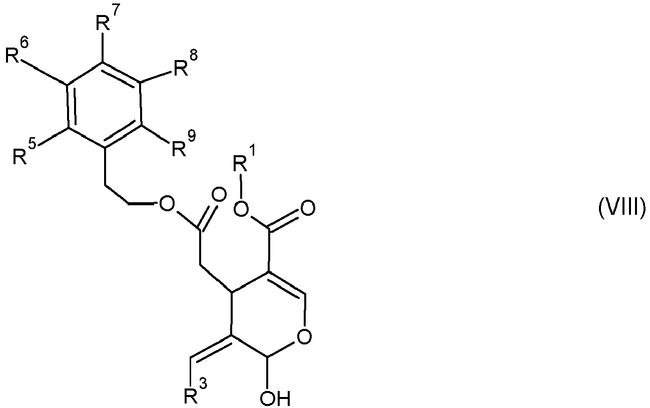

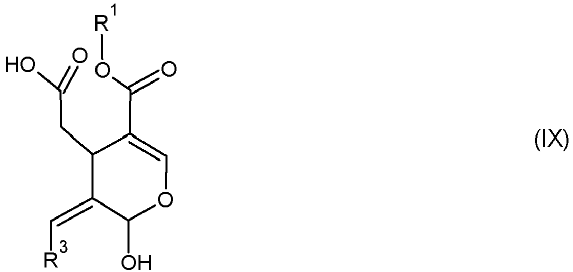

In an embodiment, residues R¹ and R³ denote CH₃ in formulae (VIII) and (IX).

In an embodiment, the cross-linking agent comprises a compound corresponding to the following formulae (X) or (XI) or corresponds itself to these formulae:

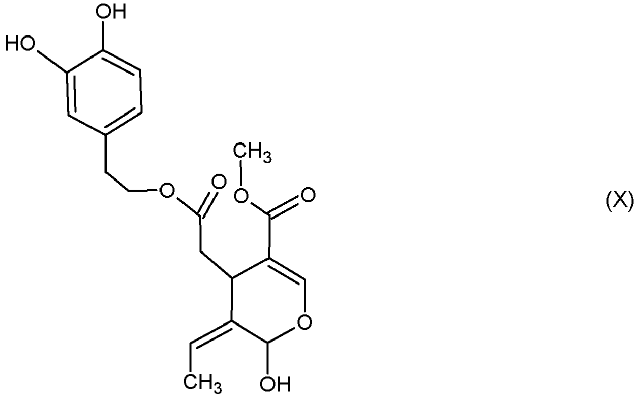

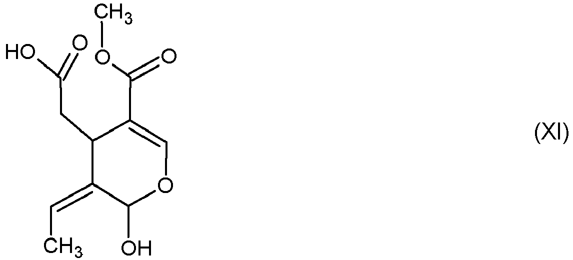

A cross-linking agent having a structure corresponding to formula (X) is present in equilibrium with a structure corresponding to the following formula (XII):

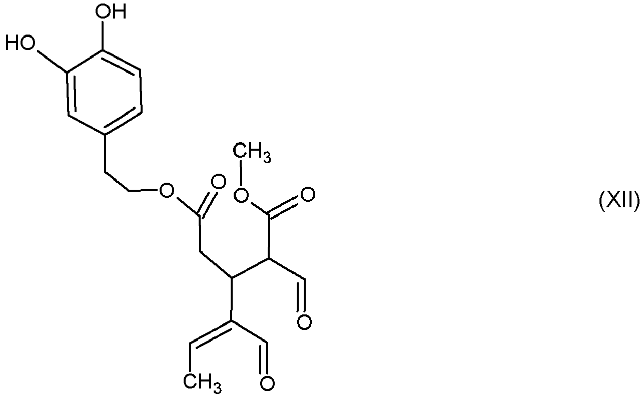

A cross-linking agent having a structure corresponding to formula (XI) is present in equilibrium with a structure corresponding to the following formula (XIII):

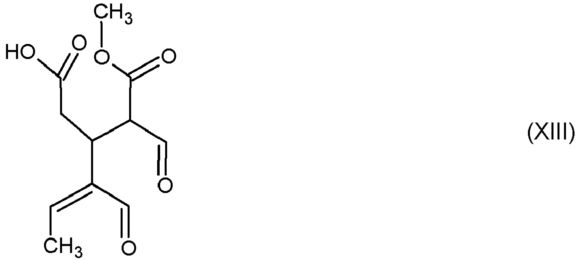

In an embodiment, the cross linking agent comprises at least one compound or is a compound according to formula (VI), to formula (X), to formula (XI), to formula (XII), to formula (XIII) or a derivative thereof.

In an embodiment, the term "derivative" denotes a compound that can be derived by a naturally occurring biotransformation process from a specific compound. I.e., these derivatives would be formed within a human or animal body due to enzymatic activity or non-enzymatic biochemical transformation or maturation processes. Specific examples of derivatives of compounds according to formula (VI), to formula (X), to formula (XI), to formula (XII), or to formula (XIII) are compounds corresponding to the following formulae (XIV) to (XXII):

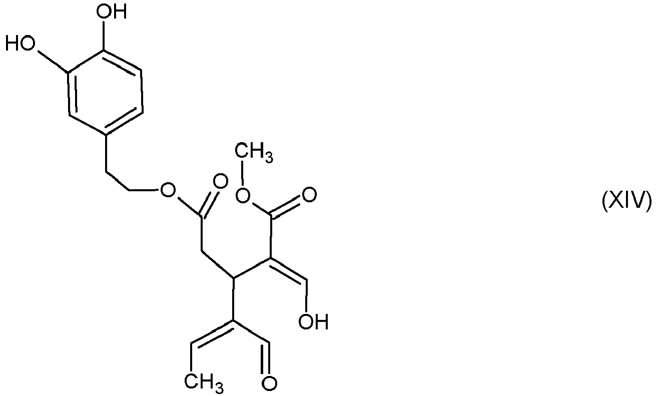

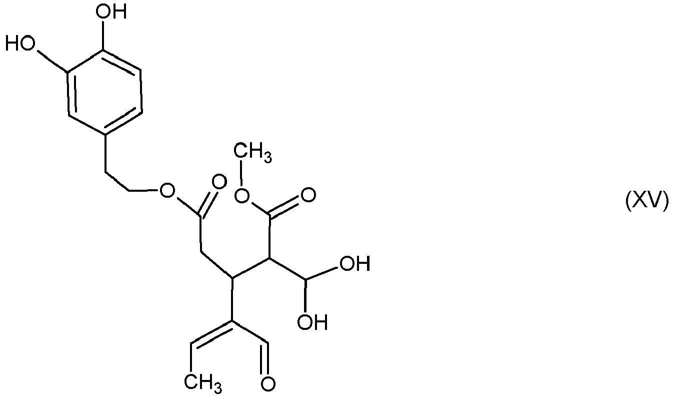

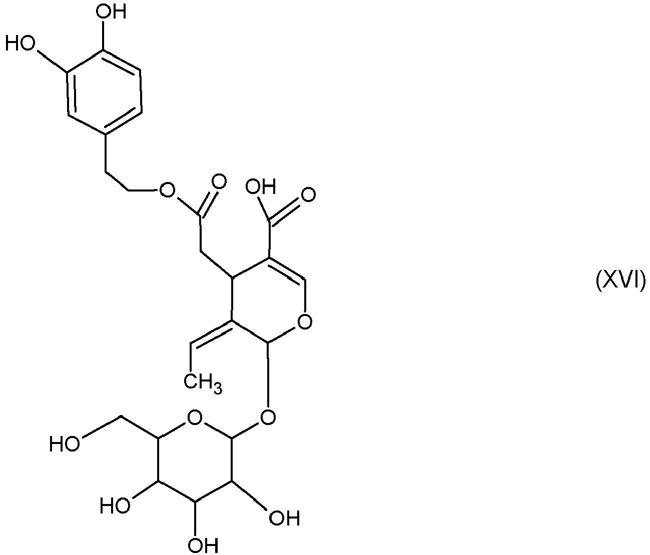

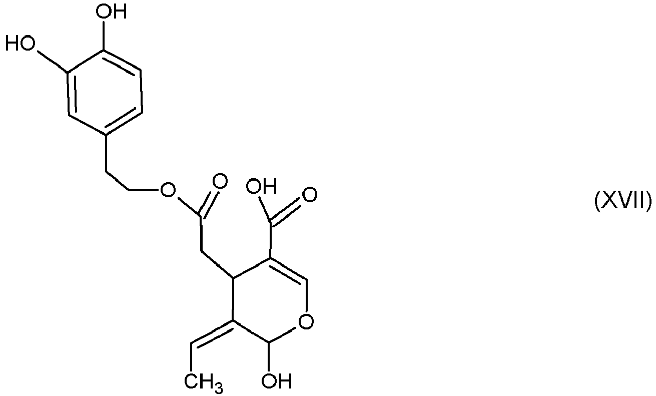

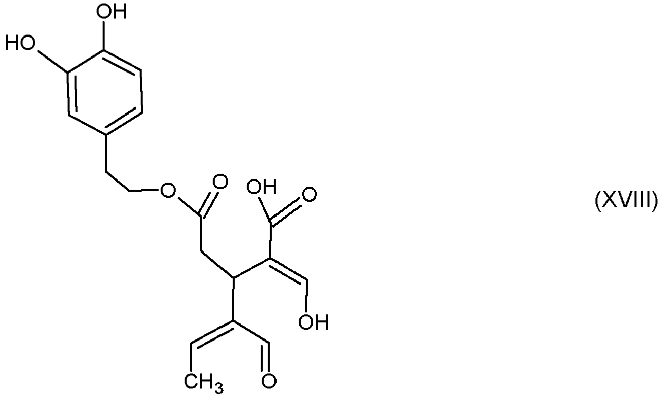

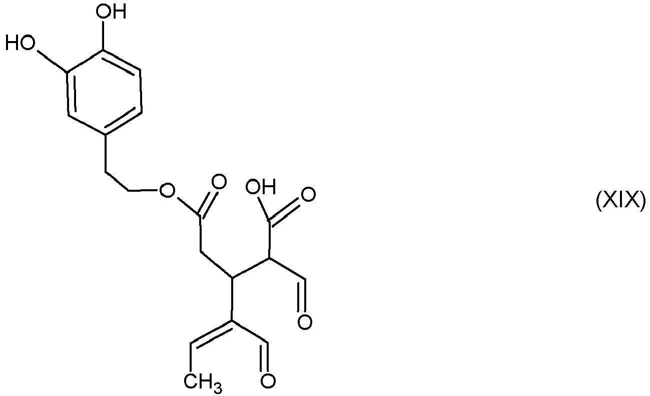

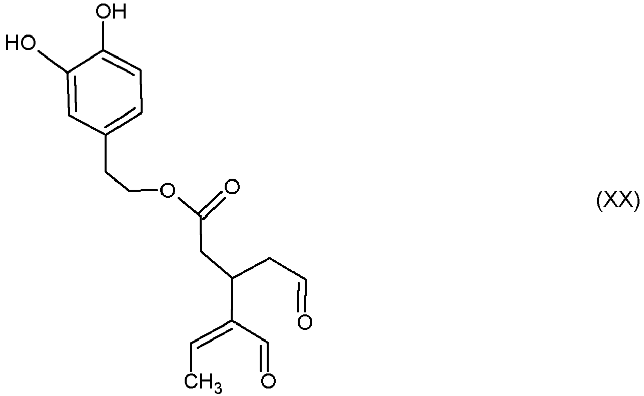

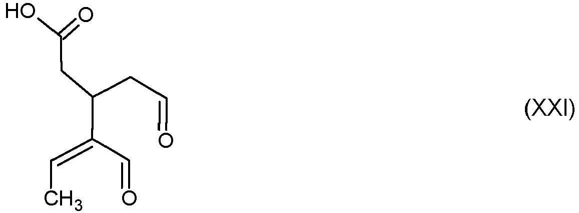

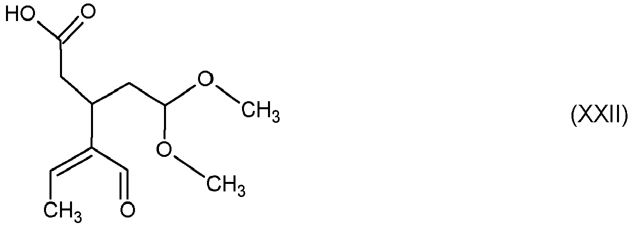

All of these derivatives, in particular compounds having a structure according to formulae (XI), (XIII), (XIX), (XX), (XXI), or (XXII) are particularly appropriate to cross-link human or animal body tissue.

Compounds that can be derived from the compound having a structure according to formula (I) in a similar biochemical way as the specific derivatives explained above are also encompassed by the presently claimed subject matter and form part of an aspect of the invention.

In an embodiment, at least two of residues R¹⁰, R¹¹, R¹², R¹³ denote OH.

In an embodiment, each of residues R¹⁰, R¹¹, R¹², R¹³ denotes OH.

In an embodiment, residues R⁵, R⁶, and R⁹ denote H, residues R⁷ and R⁸ denote OH, residues R¹ and R³ denote CH₃, residue R² corresponds to formula (III), and residues R¹⁰, R¹¹, R¹², and R¹³ denote OH. Then, the compound has a structure corresponding to the following general formula (XXIII):

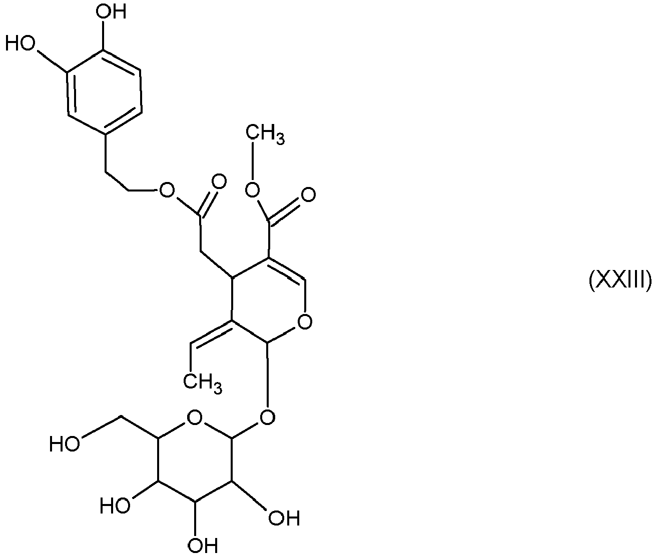

In an embodiment, residues R⁵, R⁶, and R⁹ denote H, residues R⁷ and R⁸ denote OH, residues R¹ and R³ denote CH₃, residue R² corresponds to formula (V), residues R¹⁰, R¹¹, R¹², R¹³ denote OH, and the residues of the structure according to formula (I) have a conformation according to formula (IV). Then, the compound has a structure corresponding to the following general formula (XXIV):

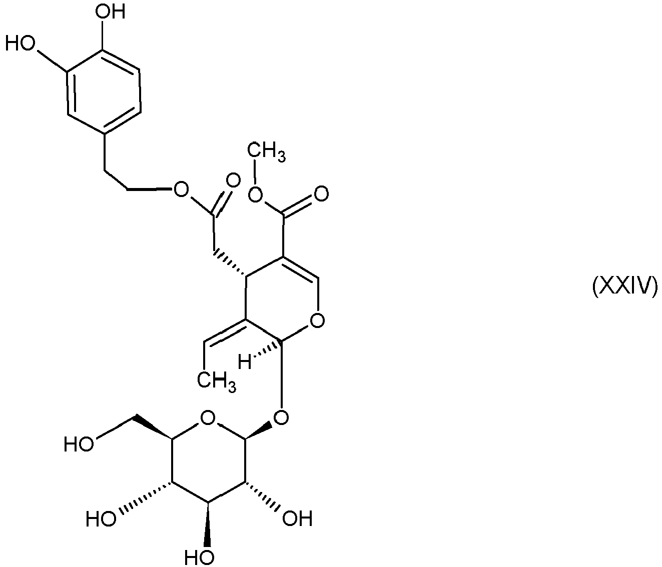

In an embodiment, the body tissue, the given shape of which is to be preserved, is cardiac tissue.

In an embodiment, the body tissue, the given shape of which is to be preserved, is cardiac muscle tissue.

In an embodiment, the body tissue, the given shape of which is to be preserved, is pericardial tissue.

In an embodiment, the cardiac valve prosthesis is an aortic valve prosthesis, a pulmonary valve prosthesis, a mitral valve prosthesis, or a tricuspid valve prosthesis.

In an embodiment, the shape of the cardiac valve, i.e. the shape to be given to the body tissue, is defined by performing the steps explained in the following. Firstly, a 3-D image of a diseased cardiac valve of an individual is obtained by an appropriate imaging method, e.g., by magnetic resonance imaging (MRI), computed tomography (CT), (3-D) ultrasound, or 3-D rotational angiography. Secondly, a 3-D reconstruction of the imaging data is performed while correcting the disease (so-called virtual valve surgery). Thirdly, a digital 3-D mold of the required cardiac valve prosthesis is generated. Fourthly, a "real" cardiac valve mold is produced on the basis of the digital 3-D mold. This can be accomplished, e.g., by 3-D printing or injection molding or other appropriate techniques.

For carrying out the shaping process, the body tissue is inserted into the cardiac valve mold and shaped by an appropriate shaping technique, e.g., by deep drawing. When the body tissue has adopted the desired form in the cardiac valve mold, the cross-linking agent is added to the shaped body tissue. The addition of the cross-linking agent results in cross-linking of the body tissue so that it stably remains in the shape that was given to the body tissue in the cardiac valve mold. An artificial cardiac valve, i.e. a cardiac valve prosthesis, results that is made from the body tissue. Due to chemical cross-linking of the body tissue, the shaped body tissue remains in its given shape even after removing the cross-linking agent and the mold. It is possible to implant the artificial cardiac valve to an individual.

In an embodiment, the cross-linking agent is used in a concentration of 0.01 to 10 % (v/v) or (w/w), in particular of 0.02 to 9 %, in particular of 0.03 to 8 %, in particular of 0.04 to 7 %, in particular of 0.05 to 6 %, in particular of 0.06 to 5 %, in particular of 0.07 to 4 %, in particular of 0.08 to 3 %, in particular of 0.09 to 2 %, in particular of 0.1 to 1 %, in particular of 0.2 to 0.9 %, in particular of 0.3 to 0.8 %, in particular of 0.4 to 0.7 %, in particular of 0.5 to 0.6, with respect to the total amount of treatment solution.

In an embodiment, the cross-linking agent is kept in contact with the shaped body tissue over a time period lying in a range from 1 hour to 72 hours, in particular from 2 hours to 48 hours, in particular from 3 hours to 36 hours, in particular from 4 hours to 24 hours, in particular from 5 hours to 20 hours, in particular from 6 hours to 15 hours, in particular from 8 hours to 12 hours.

In an embodiment, the temperature is lying in a range of from 15 °C to 40 °C, in particular from 20 °C to 38 °C, in particular from 22 °C to 37 °C, in particular from 25 °C to 35 °C, in particular from 27 °C to 30 °C during an incubation of the shaped body tissue with the cross-linking agent.

In an embodiment, a treatment solution comprising the cross-linking agent also comprises a buffering agent that is capable of buffering the treatment solution around a pH value of approximately 5. A particular appropriate pH value of the treatment solution is a pH value lying in a pH range of from pH 4 to pH 6, in particular from pH 4.5 to pH 5.5, in particular from pH 4.7 to pH 5.2, in particular from pH 4.8 to pH 5.0. A citrate buffer is a particularly appropriate buffering agent.

In an embodiment, the shaped body tissue and the treatment solution are agitated on a shaker such as a rocking shaker during at least a part of the treatment of shaping process. To give an example, an agitation can be carried out over time period lying in a range of from 5 minutes to 2 hours, in particular of from 10 minutes to 1.5 hours, in particular of from 20 minutes to 1 hour, in particular of from 30 minutes to 45 minutes. The agitation is typically carried out at the beginning of the cross-linking process. An appropriate agitation speed is a speed lying in a range of from 10 rounds per minute (rpm) to 500 rpm, in particular of from 20 rpm to 450 rpm, in particular of from 30 rpm to 400 rpm, in particular of from 40 rpm to 350 rpm, in particular of from 50 rpm to 300 rpm, in particular of from 60 rpm to 250 rpm, in particular of from 70 rpm to 200 rpm, in particular of from 80 rpm to 150 rpm, in particular of from 90 rpm to 100 rpm.

In an embodiment, the body tissue that is shaped originates from the same individual to which the cardiac valve prosthesis is afterwards implanted. Then, the cardiac valve prosthesis is an autologous cardiac valve prosthesis.

In an embodiment, the body tissue is excised from the human or animal body prior to performing the shaping process and prior to cross-linking the body tissue. Such excision is typically performed by usual surgical techniques.

In an aspect, the present invention relates to a cardiac valve prosthesis arrangement comprising a cardiac valve prosthesis according to the preceding explanations and a carrier (e.g., a stent).

In an aspect, the present invention relates to a medical method of treating a cardiac disease resulting from impaired cardiac valve by replacing an impaired or diseased cardiac valve of a human or animal in need thereof by a cardiac valve prosthesis according to the preceding explanations.

In an aspect, the present invention relates to the medical use of a cardiac valve prosthesis according to the preceding explanations in therapy of a cardiac disease resulting from an impaired cardiac valve. Such a cardiac disease can be, e.g., a cardiac valve insufficiency or a stenosis.

In an embodiment, the cardiac valve prosthesis is implanted in the same human or animal body from whom the body tissue was obtained that was used to manufacture the cardiac valve prosthesis. Then, the cardiac valve prosthesis is an autologous cardiac valve prosthesis.

In an aspect, the present invention relates to a medical method for manufacturing a cardiac valve prosthesis for a human or animal individual in need thereof. Thereby, the method comprises the following steps: providing human or animal body tissue; shaping, in a shaping process, the body tissue into a desired size and shape of a cardiac valve; and contacting the shaped body tissue with a cross-linking agent to fix and stabilize the body tissue and to thereby preserve the shape given to the body tissue by the shaping process. By these method steps, a cardiac valve prosthesis is obtained. In an embodiment, the cross-linking agent comprises or consists of a compound having a structure according to general formula (I) as explained above. Thereby, the individual residues of formula (I) have the meanings as explained above.

All embodiments of the described cardiac valve prosthesis, the described methods and the described uses can be combined in any desired way and can be transferred to the described use and the described other methods, and vice versa.

Further details of aspects of the present invention will be explained with respect to an exemplary embodiment and an accompanying Figure.

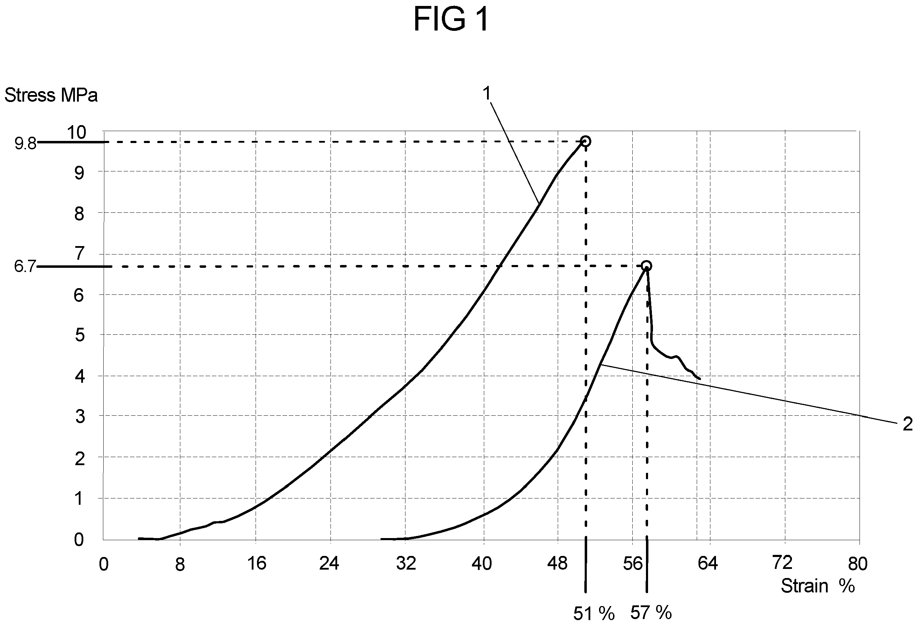

Figure 1 shows a comparison between two possible cross-linking agents used for cross-linking shaped body tissue.

To identify whether different cross-linking agents might have a different effect on the cross-linking of body tissue, in vitro tests were performed. For this purpose, human and animal body tissue was used and shaped in a deep-drawing process to give the body tissue the shape and size of a cardiac valve, namely of a pulmonary artery valve.

Afterwards, the shaped body tissue was fixated and stabilized by the addition of two different cross-linking agents. On the one hand, glutaraldehyde (GA) was used, on the other hand a compound having the structure of formula (X) was used. The latter compound will be referred to as compound X in the following. The final concentration of GA was chosen to be in a range of 0.2 to 0.625 % in the treatment solution. The final concentration of compound X was chosen to be 0.05 % in the treatment solution. The incubation was carried out over time period of 20 minutes (GA) or 24 hours (compound X) at a temperature lying in a range of 20 °C to 40 °C. The treatment solution containing the cross-linking agent was buffered by a citrate buffer in a pH range of pH 4.8 to pH 5.0. Within the first 30 minutes of the cross-linking process, the shaped body tissue and the treatment solution were agitated by a rocking shaker at 100 rpm.

Afterwards, a tensile test was carried out. While both GA and compound X were generally able to cross-link the shaped body tissue, it turned out that compound X was even more appropriate for the cross-linking process since it resulted in a more stable structure of the shaped body tissue.

As can be seen from Figure 1, the body tissue treated with compound X (curve 1) showed a 1.5-fold higher stress resistance than the body tissue cross-linked with GA (curve 2) (9.5 MPa vs. 6.7 MPa). At the same time, the maximum achieved strain was 10 % higher in case of the cross-linking with GA than in case of compound X (57 % vs. 51 %). For the functioning of a cardiac valve, however, a higher stress resistance and a sufficiently high strain resistance is believed to be more important than a high strain resistance alone. Therefore, it was decided to carry out subsequent characterization tests only with respect to the shaped body tissue cross-linked with compound X.

An investigation of the shrinking temperature by differential scanning calorimetry (DSC) showed that the body tissue was sufficiently cross-linked. Subsequent cytotoxicity and biocompatibility tests showed no relevant cytotoxicity and sufficiently high biocompatibility. When placing the cross-linked body tissue into a fibroblast culture, no necroses could be observed.

Afterwards, in vivo tests were performed to evaluate the stability of the cross-linked body tissue over an extended period of time under real conditions.

In a first preclinical study, the general feasibility and safety of the heart valve replacement method was successfully shown.

In a second preclinical study, the long-term stability of the manufactured cardiac valve prosthesis was examined. A sufficiently high stabilization of the cross-linked tissue over a time period of 1.5 years was shown in an animal model (sheep). No cardiac insufficiency with more than 20 % regurgitation fraction was observed. Furthermore, no cardiac valve stenosis could be observed.

The manufactured cardiac valve prosthesis was also subjected to different histologic examinations. The thickness, length, and structure of the manufactured valve prosthesis corresponded to the thickness, length and structure of the replaced natural heart valve. No thrombi could be observed in general and in the hinge region of the cusps. A full and correctly localized re-endothelialization was observed for the heart valve prosthesis. A correctly localized formation of neointima to a full extent could be observed.

Upon analyzing a foreign body response as well as other inflammation responses with a focus on M1 (CD80), M2 (CD163) macrophages, T cells (CD3), B cells (CD79a), an increased amount of M2 macrophages was observed. This can be taken as an indication of an immune response with desired subsequent differentiation to myofibroblasts.

No relevant neovascularization could be observed. The cardiac valve prosthesis showed full apposition onto the pulmonary arterial wall. No calcification could be observed. Furthermore, no indicators of necroses of the native pulmonary arterial wall could be seen.

Summarizing, the cardiac valve prosthesis manufactured by shaping body tissue and cross-linking it with cross-linking compound X resulted in a fully functional cardiac prosthesis that was properly integrated into the native surrounding body tissue and that remained stable over an extended period of time.

## Claims

1. Cardiac valve prosthesis, obtainable by a method comprising the following steps:
a) providing human or animal body tissue,
b) shaping the body tissue in a shaping process to give the body tissue a shape and size of a cardiac valve,
c) fixation and stabilization of the body tissue by a cross-linking agent, thereby preserving the shape given to the body tissue by the shaping process and thus obtaining a cardiac valve prosthesis.

2. Cardiac valve prosthesis according to claim 1, **characterized in that** the cross-linking agent comprises at least one compound having a structure according to general formula (I) or a salt or derivative thereof: 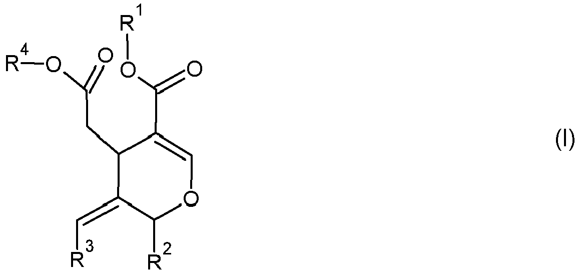 wherein
R¹ and R³ denote independently of each other and independently of other residues in the compound H or CH₃,
R⁴ denotes independently of other residues in the compound H or a residue having a structure according to general formula (II): 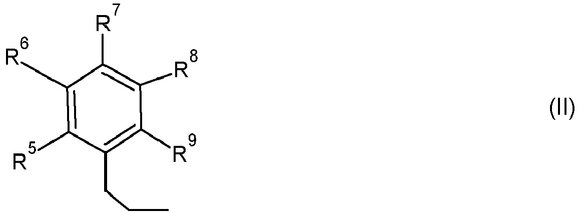
wherein
R⁵, R⁶, R⁷, R⁸, R⁹ denote independently of each other and independently of other residues in the compound H or OH,
R² denotes independently of other residues in the compound H, OH or a residue having a structure according to general formula (III): 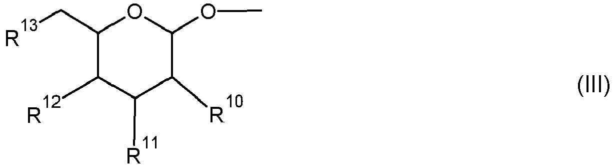
wherein
R¹⁰, R¹¹, R¹², R¹³ denote independently of each other and independently of other residues in the compound H or OH.

3. Cardiac valve prosthesis according to claim 2, **characterized in that** the residues of the structure according to formula (I) have a conformation according to formula (IV):

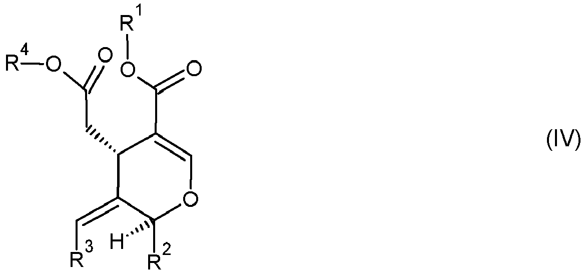

4. Cardiac valve prosthesis according to claim 2 or 3, **characterized in that** the residue R² has a structure according to general formula (III) and has a conformation according to formula (V):

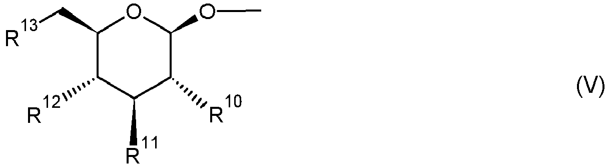

5. Cardiac valve prosthesis according to any of claims 2 to 4, **characterized in that** at least two of residues R⁵, R⁶, R⁷, R⁸, and R⁹ denote OH.

6. Cardiac valve prosthesis according to any of claims 2 to 5, **characterized in that** residues R⁵, R⁶, and R⁹ denote H and **in that** residues R⁷ and R⁸ denote OH.

7. Cardiac valve prosthesis according to any of claims 2 to 6, **characterized in that** residues R¹ and R³ denote CH₃.

8. Cardiac valve prosthesis according to any of claims 2 to 7, **characterized in that** residue R² denotes OH.

9. Cardiac valve prosthesis according to any of claims 2 to 8, **characterized in that** each of residues R¹⁰, R¹¹, R¹², R¹³ denotes OH.

10. Cardiac valve prosthesis according to any of claims 2 to 9, **characterized in that** residues R⁵, R⁶, and R⁹ denote H, **in that** residues R⁷ and R⁸ denote OH, **in that** residues R¹ and R³ denote CH₃, **in that** residue R² corresponds to formula (III), and **in that** residues R¹⁰, R¹¹, R¹², R¹³ denote OH.

11. Cardiac valve prosthesis according to claims 3 and 4, **characterized in that** residues R⁵, R⁶, and R⁹ denote H, **in that** residues R⁷ and R⁸ denote OH, **in that** residues R¹ and R³ denote CH₃, **in that** residue R² corresponds to formula (V), **in that** residues R¹⁰, R¹¹, R¹², R¹³ denote OH, and **in that** the residues of the structure according to formula (I) have a conformation according to formula (IV).

12. Cardiac valve prosthesis according to any of claims 2 to 11, **characterized in that** the body tissue is connective tissue, fascial tissue, peritoneal tissue or cardiac tissue.

13. Cardiac valve prosthesis according to claim 12, **characterized in that** the body tissue is pericardial tissue.

14. Cardiac valve prosthesis according to any of the preceding claims for use in therapy of a cardiac disease resulting from an impaired cardiac valve.

15. Cardiac valve prosthesis according to any of claims 1 to 13 for use according to claim 14, wherein the cardiac valve prosthesis is implanted in the same human or animal body from whom the body tissue was obtained that is used to manufacture the cardiac valve prosthesis.
